# EUROPEAN PATENT APPLICATION

(11) **EP 2 322 159 A1**
(43) Date of publication of application: **18.05.2011**
(21) Application number: 09174717.0
(22) Date of filing: 30.10.2009
(51) Int. Cl.: A61K 31/05

(54) **Use of pterostilbene (pter) as medicament for prevention and/or treatment of skin diseases, damages or injures or as cosmetic**

(71) Applicant: Green Molecular, S.L., 46980 Valencia (ES)
(72) Inventor: Jose María, Estrela Ariquel, 46980, Valencia (ES); Miguel A., Asensi Miralles, 46980, Valencia (ES)
(74) Representative: Illescas, Manuel

(57) **Abstract**

The present invention refers to the use of PTER, or any acceptable salt thereof, optionally in combination with QUER, or any acceptable salt thereof, for the manufacture of pharmaceutical compositions for preventing and/or treating skin diseases, damages or injuries. Those methods of prevention and/or treatment comprise the administration of an effective amount of a composition comprising PTER as active compound, or any acceptable salt thereof, optionally in combination with QUER, or any acceptable salt thereof, to the subject. PTER, or any acceptable salt thereof, optionally in combination with QUER, or any acceptable salt thereof, can be also used for manufacturing cosmetic compositions, preferably sunscreen cosmetics. The present invention also refers to the topical compositions (pharmaceuticals or cosmetics), per se, comprising PTER as active compound, or any acceptable salt thereof, optionally in combination with QUER, or any acceptable slat thereof, characterized by being formulated in the form of liposomes, and to the method for preparing said liposome formulations.

## Description

### FIELD OF THE INVENTION

The present invention may be encompassed in the pharmaceutical or cosmetic technical fields.

### STATE OF THE ART

A high intake of fruits and vegetables is associated with a healthy lifestyle. This association is due to the presence in food of natural antioxidants, mainly polyphenolic compounds. One of the polyphenols that has arisen more interest in the scientific community is resveratrol (3,4',5-trihydroxy-trans-stilbene; RESV), a small polyphenol present in significant amounts in wine, to which potential beneficial effects have been attributed in different chronic diseases (especially cancer, cardiovascular disease, type II diabetes and neurodegenerative diseases). The anticancer properties of RESV were first proposed by Jang et al. (Jang M, Cai L, Udeani GO, Slowing KV, Thomas CF, Beecher CW, Fong HH, Farnsworth NR, Kinghorn AD, Mehta RG, Moon RC, Pezzuto JM. (1997) Science275(5297):218-20), with effects on the three stages of cancer development (initiation, promotion and progression). The anticancer effects of RESV are very limited by its low bioavailability (Asensi M, Medina I, Ortega A, Carretero J, Bano MC, Obrador E, Estrela JM. (2002) Free adic Biol Med. 33(3): 387-98). Due to that drawback, some other polyphenols with improved bioavailabilty were assayed for cancer treatment. The combination of pterostilbene (3,5-dimethoxy-4 '-hydroxy-trans-resveratrol, PTER, a derivative of RESV) and quercetin (3,3', 4 ',5,6-pentahidroxiflavona; QUER), two small natural polyphenols, having the following formula: 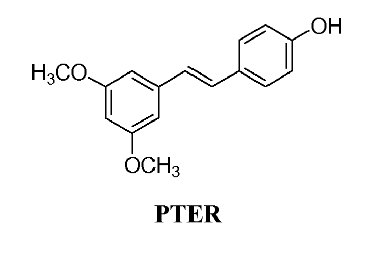 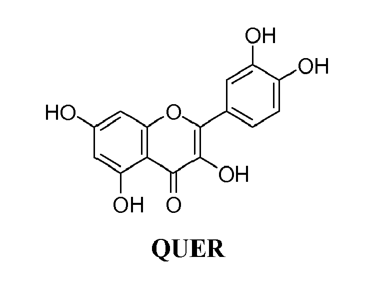 was shown to be effective to inhibit metastatic growth of a highly aggressive murine malignant melanoma (Ferrer P, Asensi M, Segarra R, Ortega A, Benlloch M, Obrador E, Varea MT, Asensio G, Jorda L, Estrela JM. (2005) Neoplasia. 7(1):37-47)*.* Furthermore the association of PTER and QUER, by enhancing the antitumor effects of chemotherapy plus radiotherapy, facilitates the elimination of resistant human colorectal cancer xenografted in immunodeficient mice (Priego S, Feddi F, Ferrer P, Mena S, Benlloch M, Ortega A, Carretero J, Obrador E, Asensi M, Estrela JM. (2008) Mol Cancer Ther. 7(10): 3330-42)*.* PTER and QUER were administered intravenously and/or orally.

Recent epidemiological studies have shown that over 90% of all skin cancers are caused by excessive exposure to sun, especially ultraviolet B (UVB). The incidence of these cancers, both melanoma and non melanoma type, is increasing in a spectacular way (deaths from skin cancer have doubled in the last 20 years).

Therefore, there is a long-felt need in the art for compositions able to prevent and/or treat skin diseases (including skin cancer), injures or damages caused by prolonged exposures to radiation, particularly UVB (ultraviolet B).

However, skin diseases, injures or damages, may be caused, not only by radiation exposure, but also being due to another factors, as immune factors. Most of skin diseases, injures or damages share the presence of inflammatory processes associated to skin pathology. Moreover, skin exposure to radiation or skin inflammatory processes associated either to said radiation exposure or to other causes, as autoimmune skin diseases or allergic skin diseases, such a psoriasis, allergic contact dermatitis or atopic dermatitis, also share an increase in the oxidative stress of the skin, by the production of oxygen radicals and another oxidative species as nitrogen oxide (NO) of peroxinitrite, as a way of example.

QUER has been disclosed (Rubia Casagrande, Sandra R. Georgetti, Waldiceu A. Verri Jr., Daniel J. Dorta, Antônio C. dos Santos, Maria J. V. Fonseca [2006]; J. Photochem Photobiol B: Biology 84: 21-27 and Alena Svobodová, Jitka Psotová, Daniela Walterová [2003]; Biomed. Papers 147(2): 137-145) to be effective in the prevention of the deleterious effects of UV radiation on the skin. However, the absorption spectrum of QUER shows most of its maximum peaks at wavelengths in the range of UVA (320-400 nm), not in the range ofUVB (290-320 nm), precisely wherein the absorption spectrum of PTER has its maximum area.

Present invention gives a new step forward, toward the solution of the above problem, developing compositions focused on the prevention and/or in the treatment of skin diseases, injures or damages, based on PTER as main active compound. Moreover, present invention can be also applied to mammal (human) skin under a cosmetic formulation, particularly sunscreen, after-sun, anti-aging, anti-wrinkles, formulations, with photoprotective purposes, before any skin disease, damage or injure, might come out.

### DESCRIPTION OF THE INVENTION

The present invention mainly refers to the use of PTER, or any acceptable salt thereof, optionally in combination with QUER, or any acceptable salt thereof, for the manufacture of pharmaceutical compositions for preventing and/or treating skin diseases, damages or injuries. Moreover, the invention also relates to the use of PTER, or any acceptable salt thereof, optionally in combination with QUER, or any acceptable salt thereof, for the manufacture of cosmetics compositions for the skin. Therapeutics methods of prevention and/or treatment of skin diseases, damages or injuries, comprise the administration of an effective amount of a composition comprising PTER as active compound, or any acceptable salt thereof, optionally in combination with QUER, or any acceptable salt thereof, to a subject in need of. The present invention also refers to the compositions (pharmaceuticals or cosmetics), per se, comprising PTER as active compound or any acceptable salt thereof, optionally in combination with QUER, preferably being in the form of topical formulations and more preferably in the form of liposomes; the invention also covers the process for preparing said liposome formulations.

QUER may be replaced by any other polyphenol, providing that the combination with PTER shows synergistic effect.

For the purpose of present invention, polyphenols are a group of chemical substances which might be found in plants, but not exclusively, characterized by the presence of more than one phenol unit or building block per molecule. Polyphenols are generally divided into hydrolyzable tannins (gallic acid esters of glucose and other sugars) and phenylpropanoids, such as lignins, flavonoids, and condensed tannins.

The action of PTER and, optionally, also its combination with QUER, would be exerted in a multiple way:
a) As UV filter, because both polyphenols have chemical structures which allow radiation absorption.
b) As oxygen free radical scavengers of Reactive Oxygen Species (ROS) produced during irradiation and/or during inflammatory processes.
c) As anti-inflammatory and immunomodulatory agents.

In a preferred embodiment the compositions of the invention comprise a combination of the two active compounds PTER and QUER, or any acceptable salt thereof. Therefore, the methods of prevention and/or treatment comprise the administration of an effective amount of a composition comprising both PTER and QUER, or any acceptable salt thereof, to a subject in need of it.

A "subject", as defined in present specification, is a mammal, e.g., a human.

Inflammatory processes, as defined in present invention comprise process of acute or chronic inflammation initiated by cells already present in all tissues, mainly resident macrophages, dendritic cells, histiocytes, Kuppfer cells and mastocytes. At the onset of an infection, burn, or other injuries, these cells undergo activation and release inflammatory mediators responsible for the clinical signs of inflammation. Vasodilation and its resulting increased blood flow causes the redness and increased heat. Increased permeability of the blood vessels results in an exudation (leakage) of plasma proteins and fluid into the tissue (oedema), which manifests itself as swelling. Some of the released mediators such as bradykinin increase the sensitivity to pain (hyperalgesia). The mediator molecules also alter the blood vessels to permit the migration of leukocytes, mainly neutrophils, outside of the blood vessels (extravasation) into the tissue. The neutrophils migrate along a chemotactic gradient created by the local cells to reach the site of injury. The loss of function is probably the result of a neurological reflex in response to pain.

In addition to cell-derived mediators, several acellular biochemical cascade systems consisting of preformed plasma proteins act in parallel to initiate and propagate the inflammatory response. These include the complement system activated by bacteria, and the coagulation and fibrinolysis systems activated by necrosis, e. g. a burn or a trauma.

The acute inflammatory response requires constant stimulation to be sustained. Inflammatory mediators have short half lives and are quickly degraded in the tissue. Hence, inflammation ceases once the stimulus has been removed.

The compositions of the invention may comprise any "acceptable salt" of the active compounds included therein. As used herein, said expression means those salts of compounds of the invention that are safe and effective for use in mammals, as pharmaceuticals or as cosmetics, and that possess the desired biological activity. Illustrative salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate, and pamoate salts. The "acceptable salts" of present invention are preferably prepared from a polyphenol compound having an acidic functional group, such as a carboxylic acid functional group, and a acceptable inorganic or organic base. Suitable bases include, but are not limited to, hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxy-substituted mono-, di-, or tri-alkylamines, dicyclohexylamine; tributyl amine; pyridine; N-methyl, N-ethylamine; diethylamine; triethylamine; mono-, bis-, or tris-(2-hydroxy substituted lower alkylamines), such as mono-; bis-, or tris-(2-hydroxyethyl)amine, 2-hydroxy-tert-butylamine, or tris-(hydroxymethyl)methylamine, N,N-di-lower alkyl-N-(hydroxy lower alkyl)-amines, such as N,N-dimethyl-N-(2-hydroxyethyl)amine or tri-(2-hydroxyethyl)amine; N-methyl-D-glucamine; and amino acids such as arginine, lysine, and the like. The term "acceptable salt" also includes a hydrate of a polyphenol compound.

An excipient, as defined in present specification, is an inactive substance used as a carrier or vehicle for the active compounds (PTER and/or QUER) comprised in the composition. The compositions of the invention may comprise any excipient, preferably suitable for being included in topical compositions (i.e. dermatological acceptable excipient). The following auxiliary agents mentioned below can independent of each other be present in the composition of the invention: gelling agents, oils, waxes, thickening agents, hydrophilic or hydrophobic polymers, emulsifying agents, emollients, fatty acids, organic solvents, antioxidants, stabilizers, sequestering agents, acidifying or basifying agents, emulsifiers, emollients, surfactants, film formers, biological additives to enhance performance and/or consumer appeal such as amino acids, proteins, vanilla, aloe extract or bioflavinoids, buffering agents, chelating agents such as ethylenediaminetetra-acetic acid (EDTA) or oxalic acid, colorants, dyes, propellants, antifoaming agents, wetting agents, vitamins, emulsion stabilizers, pH adjusters, thickening agents, fragrances, preservatives, opacifying agents, water and/or alcohols. The aforementioned auxiliary agents for the composition of the invention are used in the usual amounts known by those skilled in the art.

Suitable oils for the compositions of the invention are selected from animal or vegetable or synthetic oils. Particularly preferred oils are selected from the group comprising liquid petrolatum, liquid paraffin, volatile and non-volatile silicone oils, isoparaffins, polyalphaolefins, fluorated and perfluorated oils.

Suitable stabilizers for the compositions of the invention can be of non-ionic, anionic, cationic and amphiphilic nature. Preferred stabilizers are selected from the group comprising polyethylenglycol (PEG) and derivatives thereof, tweens, tritons, spans, polygycerines, polyalkyl glycerides, alkyl sulfonates, aryl sulfonates, alkyl phosphates, derivatives of alkyl-betaine and phosphatidylglycerole.

Emulsifiers are preferably used in the compositions of present invention in amounts effective to provide uniform blending of ingredients of said compositions. Suitable emulsifiers include anionics such as fatty acid soaps, e.g., potassium stearate, sodium stearate, ammonium stearate, and triethanolamine stearate; polyol fatty acid monoesters containing fatty acid soaps, e.g., glycerol monostearate containing either potassium or sodium salt; sulfuric esters (sodium salts), e.g., sodium lauryl 5 sulfate, and sodium acetyl sulfate; and polyol fatty acid monoesters containing sulfuric esters, e.g., glyceryl monostearate containing sodium lauryl surfate; (ii) cationics chloride such as N(stearoyl colamino formylmethyl) pyridium; N-soya-N-ethyl morpholinium ethosulfate; alkyl dimethyl benzyl ammonium chloride; diisobutylphenoxytheoxyethyl dimethyl benzyl ammonium chloride; and acetyl pyridium chloride; and (iii) nonionics such as polyoxyethylene fatty alcohol ethers, e.g., monostearate; polyoxyethylene lauryl alcohol; polyoxypropylene fatty alcohol ethers, e.g., propoxylated oleyl alcohol; polyoxyethylene fatty acid esters, e.g., polyoxyethylene stearate; polyoxyethylene sorbitan fatty acid esters, e.g., polyoxyethylene sorbitan monostearate; sorbitan fatty acid esters, e.g., sorbitan; polyoxyethylene glycol fatty acid esters, e.g., polyoxyethylene glycol monostearate; and polyol fatty acid esters, e.g., glyceryl monostearate and propylene glycol monostearate; and ethoxylated lanolin derivatives, e.g., ethoxylated lanolins, ethoxylated lanolin alcohols and/or ethoxylated cholesterol.

Emollients may be also used in the compositions of the invention in such amounts to prevent or relieve dryness. Suitable emollients include, without limitation hydrocarbon oils and waxes; silicone oils; triglyceride esters; acetoglyceride esters; ethoxylated glyceride; alkyl esters; alkenyl esters; fatty acids; fatty alcohols; fatty alcohol ethers; etheresters; lanolin and derivatives; polyhydric alcohols (polyols) and polyether derivatives; polyhydric alcohol (polyol) esters; wax esters; beeswax derivatives; vegetable waxes; phospholipids; sterols; and/or amides.

Surfactants can further be used too in the compositions of present invention. Suitable surfactants are for example those surfactants generally grouped as cleansing agents, emulsifying agents, foam boosters, hydrotropes, solubilizing agents, suspending agents and non-surfactants, which facilitate the dispersion of solids in liquids.

Film formers which may be preferably used in the compositions of present invention should keep the composition smooth and even and are preferably, without limitation. Suitable film formers are selected from the group comprising acrylamide/sodium acrylate copolymer; ammonium acrylates copolymer; Balsam Peru; cellulose gum; ethylene/maleic anhydride copolymer; hydroxyethylcellulose; hydroxypropylcellulose; polyacrylamide; polyethylene; polyvinyl alcohol; pvm/MA copolymer (vinyl methylether/maleic anhydride copolymer); PVP (polyvinylpyrrolidone); maleic anhydride polymer, vinylpyrrolidon/hexadecene copolymer; acryliclacrylate copolymer and the like.

pH adjusters may also be used in the compositions of present invention. These pH adjusters are preferably selected from: ammonium hydroxide, triethanolamine or citric acid.

Thickening agents used for the compositions of the invention preferably are selected from: candelilla, carnauba, and microcrystalline waxes, crosslinked acrylic-acid polymers, carbomer, methylhydroxyethylcellulose, hydroxypropylmethylcellulose or hydroxyethylcellulose and polyethylene thickeners.

Examples of preferred organic solvents for the compositions of present invention include lower aliphatic alcohols and polyols.

Suitable antioxidants for the compositions used in present invention are preferably selected from the group comprising ascorbic acid (vitamin C), sodium-L-ascorbate, calcium-L- ascorbate, ascorbyl palmitate, butylhydroxyanisole, butylhydroxytoluene, calcium- disodium-EDTA, isoascorbic acid, lecitine, lactic acid, polyphosphate, tocopherol (vitamin E), like [alpha]-tocopherol, [gamma]-tocopherol, [delta]-tocopherol, propylgallate, octylgallate, dodecylgallate, sodium-isoascorbate, citric acid, sodium citrate, potassium citrate and tin-11-chloride.

Gelling agents, which are preferably used in the compositions of the invention, can be natural or synthetic polymers. Natural polymers are preferably selected from: Agar-Agar, alginate, pectin, carbomer, carrageenan, casein, dextrine, gelatine, arabic gum, keratine, locust bean gum, xanthan gum and the like. Preferred synthetic polymers which can be used in the compositions of the invention are selected from: acylic acid polymers, polyacryl amides and alkylene oxide polymers.

Any route of administration can be used for administering the composition of the invention to the subject, being the preferred routes of administration intravenous, oral and topical. In a preferred embodiment the composition of the invention is a topical formulation that may be formulated in liquid or in semi-solid form, preferably as liquid, fluid, foam, cream, gel, paste, balsam, spray, ointment, lotion, conditioner, tonic, milk, mousse, emulsion, serum, oil, stick, shampoo, jelly, suspension, dispersion, lacquer, paint, elixir, drop or aerosol. In a particular embodiment, the active compounds of the invention were administered topically in a liposomal preparation as shown in **Example 5**. As used in the present invention, a "liposome" is an artificial vesicle that is composed of one or more concentric layers and is used especially to deliver substances (i.e. PTER and/or QUER) to the body cells (see **Examples 4** and **5**).

Topical administration of the composition of the invention leads to a high bioavailability of the active compounds PTER and/or QUER, decreasing the amount of the compositions of the invention that produces a therapeutic response in 50% of the subjects to whom are administered, i.e. decreasing the effective dosage (ED50). In pharmacology, bioavailability is used to describe the fraction of an administered dose of unchanged drug that reaches the systemic circulation, one of the principal pharmacokinetic properties of drugs. Therefore, the bioavailability is one of the essential tools in pharmacokinetics, and it must be considered when calculating dosages for non-intravenous routes of administration.

Any skin disease, damage or injury, including skin cancer, can be prevented and/or treated with the compositions of the invention. In a preferred embodiment of the invention the skin diseases, damages or injuries are associated to inflammatory processes. Therefore the invention, in a preferred embodiment, is focused on preventing and/or treating subjects predisposed to or afflicted with an inflammatory dermatosis, comprising the administration of the composition of the invention to the affected skin area (lips, face or skin in general) and/or by any other alternative way of administration. The term "inflammatory dermatosis" includes a range of skin disorders, including, but not limited to, sebaceous gland disorders, papulosquamous dermatoses, allergic dermatoses, pruritic dermatoses, vascular dermatoses, bacterial dermatoses, viral dermatoses, mycolic skin infections, granulomatous dermatoses, parasitic skin dermatoses, exfoliative dermatitis, bullous dermatoses, pigmented dermatoses, photosensitive dermatoses, dermatoses caused by collagen diseases, and dermatoses due to internal diseases. The inflammatory dermatosis can also be associated with an autoimmune condition, in which case it is referred to herein as "autoimmune dermatosis."

In a preferred embodiment of the invention, the inflammatory dermatosis is a sebaceous gland disorder, e.g., an acneiform disorder such as acne vulgaris, acne conglombata, hidradenitis suppurativa, acne rosacea, seborrhea, seborrheic dermatitis, gram negative folliculitis, pyoderma faciale, steatocystoma multiplex, sebaceous hyperplasia, or rhinophyma. In a more preferred embodiment of the invention, the composition/formulation comprising PTER or any acceptable salt thereof, optionally in combination with QUER or any acceptable salt thereof , is used to treat acne vulgaris. As is well known, acne vulgaris is a chronic skin condition characterized by comedones and papules, and can be quite severe; in particularly severe cases, pustules, cysts, and permanent scarring may occur.

In still another preferred embodiment of present invention, the inflammatory dermatosis prevented and/or treated is a papulosquamous dermatosis such as, for example, psoriasis, Pityriasis rosea, tinea versicolor, or lichen planus. The method and formulations of the invention are particularly useful in treating psoriasis, an autoimmune inflammatory disorder that has proven difficult to treat with conventional agents.

In a further preferred embodiment of present invention, the inflammatory dermatosis treated is an autoimmune dermatosis that may be, by way of example, atopic dermatitis, mast cell disease, bullous pemphigoid, pemphigus vulgaris, necrotizing vasculitis, discoid lupus erythematosus, systemic lupus erythematosis, or dermatitis herpetiformis.

In other embodiments, examples of the various types of inflammatory dermatosis with which the method and formulation of the invention are effective are as follows:
- Allergic Dermatoses: contact dermatitis; photoallergic dermatitis; industrial dermatoses caused by exposure to a variety of compounds used by industry that are contact irritants; atopic eczema (infantile and adult); and dermatoses caused by drugs and nummular eczema.
- Pruritic Dermatoses: winter, senile, and essential pruritus; pruritus ani; eternal otitis; pruritis hiemalis; pruritis vulvae; and pruritus scrotae.
- Vascular Dermatoses: erythema multiforme; erythema nodosum; stasis dermatitis; purpuric dermatoses such as those associated with thrombocytopenic purpura, nonthrombocytopenic purpura, dysproteinemic purpura, actinic purpura, scorbutic purpura, and Henochs purpura; ecchymoses; stasis purpura; primary and secondary telangiectases.
- Bacterial Dermatoses: pyoderma such as impetigo, ecthyma, folliculitis, furuncles styes, carbuncles, sweat gland infections, erysipelas, erythrasma, infected ulcers, and infected eczematoid dermatitis; and bacterial dermatoses associated with systemic bacterial infections such as scarlet fever, granuloma inguinale, chancroid, tuberculosis, leprosy, gonorrhea, rickettsial diseases, actinomycosis, and syphilis.
- Viral Dermatoses: such as those caused by Herpes simplex virus, Kaposi's varicelliform eruption, zoster, chickenpox, smallpox, vaccinia, molluscum contagiosum, lymphogranuloma venereum, exanthematous diseases such as German measles, roseola, and erythema infectiosum.
- Mycolic Skin Infections: tinea cruris (superficial fungal infections of the skin in various body sites); athlete's foot (dermatophytosis of the feet caused to infection with trichophyton mentagrophytes); tinea unguium (onychomycosis); sporotrichosis; coccidioidomycosis; histoplasmosis; and North American blastomycosis.
- Granulomatous Dermatoses: sarcoidosis; granuloma annulare; reticulohistiocytoma; and silica-induced granulomas.
- Parasitic Skin Infections: scabies, cheyletiella dermatitis; demodicosis; pediculosis
- Pigmented Dermatoses: Chloasma (melasma) and vitiligo.
- Collagen Diseases: scleroderma and dermatomyositis.
- Dermatoses Due to Internal Diseases: pyoderma gangrenosum associated with ulcerative colitis, and ulcers due to diabetes.
- Photosensitive Dermatoses: Exogenous types such as drug-induced photodermatitis and contact dermatitis with photoallergic components; and endogenous types such as those associated with porphyrias and polymorphous light eruptions.

In a preferred embodiment the skin diseases, damages or injuries are caused by the exposure to radiation, either acute or chronic. Then the disease is selected among: skin cancer, melanoma, polymorphous light eruption, actinic keratosis, solar urticaria, xeroderma pigmentosum, photoageing, chronic actinic dermatitis or sunburn. The term "radiation" comprises any kind of radiation able to cause injuries or damages in the skin, for example the radiotherapy used for treating cancer or ultraviolet radiation (UV), preferably UVB emitted by the sun.

The term "prevention" as used in the present invention means to avoid fully or partially, the appearance of skin physiopathologies associated to oxidation stress, excessive exposure to radiation, particularly to UV radiation linked to body-tanning , in particular to UVB radiation attributed to sun-tanning, allergy, immune responses and inflammatory processes.

As used in the present invention, the term "photo-protection" refers to the administration of the composition of the invention before the subject is exposed to radiation, mainly UVB radiation, decreasing or mitigating the risk of suffering damages. Therefore those term "photo-protection" are understood in the present invention as being comprised under the term "prevention".

On the other hand, the term "treatment" is linked to the administration of the composition of the invention after the subject has suffered the symptoms of skin disease, damage or injure. As a way of example, the term "treatment" is preferably applied to the administration of any of the compositions disclosed in present invention to a person which has been exposed to radiation, mainly UVB radiation, repairing the damaged parts of the skin and restoring its original function and integrity. Moreover term "treatment" is linked to the administration of the composition of the invention to a subject suffering any skin disease, damage or injury associated to inflammatory processes.

PTER, optionally with QUER, or any acceptable salt thereof, may be formulated in combination with other active compounds. Among those other active compounds analgesics, anti-inflammatory agents, anti-allergics, immunomodulators, healing agents and radiation filters are preferred. For the list of UV filters suitable for being ingredients of the pharmaceutical or cosmetic formulations of present invention accompanying to PTER and, optionally, to QUER, or any acceptable salts thereof, we refer to the UV absorbing substances (A and/or B) disclosed in WO 2008067928. Most preferred UV filters are selected from:

**Table 1**

| **UV-filter** | **Other names** |
|---|---|
| p-Aminobenzoic acid | PABA |
| Padimate O | OD-PABA, octyldimethyl-PABA, σ-PABA |
| Phenylbenzimidaz ole sulfonic acid | Ensulizole, Eusolex 232, PBSA, Parsol HS |
| Cinoxate | 2-Ethoxyethyl p-methoxycirmamate |
| Dioxybenzone | Benzophenone-8 |
| Oxybenzone | Benzophenone-3, Eusolex 4360, Escalol 567 |
| Homosalate | Homomethyl salicylate, HMS |
| Menthyl anthranilate | Meradimate |
| Octocrylene | Eusolex OCR, 2-cyano-3,3diphenyl acrylic acid, 2-ethylhexylester |
| Octyl methoxycinnamate | Octinoxate, EMC, OMC, Ethylmethoxycinnamate, Escalol 557, 2-ethylhexyl-paramethoxycinnamate, Parsol MCX |
| Octyl salicylate | Octisalate, 2-Ethylhexyl salicylate, Escalol 587, 2-Hydroxy-4-Methoxybenzophenone-5-sulfonic acid, |
| Sulisobenzone | 3-benzoyl-4-hydroxy-6-methoxybenzenesulfonic acid, Benzophenone-4, Escalol 577 |
| Trolamine salicylate | Triethanolamine salicylate |
| Avobenzone | 1-(4-methoxyphenyl)-3-(4-tert-butylphenyl)propane-1,3-dione, Butyl methoxy dibenzoylmethane, BMDBM, Parsol 1789, Eusolex 9020 |
| Ecamsule | Mexoryl SX, Terephthalylidene Dicamphor Sulfonic Acid |
| Titanium dioxide | CI77891 |
| Zinc oxide | |
| 4- Methylbenzylidene camphor | Enzacamene, Parsol 5000, Eusolex 6300, MBC |
| Tinosorb M | Bisoctrizole, Methylene Bis-Benzotriazolyl Tetramethylbutylphenol, MBBT |
| Tinosorb S | Bis-ethylhexyloxyphenol methoxyphenol triazine, Bemotrizinol, BEMT, anisotriazine |
| Neo Heliopan AP | Bisdisulizole Disodium, Disodium phenyl dibenzimidazole tetrasulfonate, bisimidazylate, DPDT |
| Mexoryl XL | Drometrizole Trisiloxane |
| Benzophenone-9 | Uvinul DS 49, CAS 3121-60-6, Sodium Dihydroxy Dimethoxy Disulfobenzophenone [61] |
| Uvinul T 150 | Octyl triazone, ethylhexyl triazone, ET |
| Uvinul A Plus | Diethylamino Hydroxybenzoyl Hexyl Benzoate |
| Uvasorb HEB | Iscotrizinol, Diethylhexyl butamido triazone, DBT |
| Parsol SLX | Dimethico-diethylbenzalmalonate, Polysilicone-15 |
| Isopentenyl-4-methoxycinnamate | |

Several techniques were carried out in the present invention, such as the determination of the skinfold or epidermis thickness, both after and before the exposure to radiation, in order to elucidate the effectiveness or efficiency of the compositions of the invention in preventing and/or treating skin diseases, lesions or damages. All those techniques are well-known in the art and pertain to the common general knowledge. Moreover, both techniques are routinely used for testing the ability of different compounds to prevent or treat skin diseases, damages or injuries. The smaller the thickness or the folds of the epidermis, the more effective the treatment is (see **Examples 10**, **11** and **12**). Such as it can be seen in the Examples active compound PTER, or any acceptable salt thereof, can be used, for the manufacture of a topical composition for preventing and/or treating skin diseases, damages or injuries. Moreover, the combination of active compounds PTER and QUER, or any acceptable salt thereof, shows a synergistic effect improving the function of each active compound taken separately in some of the assays as explained below. Therefore, the term "synergistic" when used in connection with the present invention means that the overall therapeutic effect of a combination of PTER and QUER, or any acceptable salt thereof, when administered as combination therapy for preventing and/or treating skin diseases, damages or injuries caused by the exposure to radiation, is greater than the therapeutic effects of these active compounds when each one is administered independently.

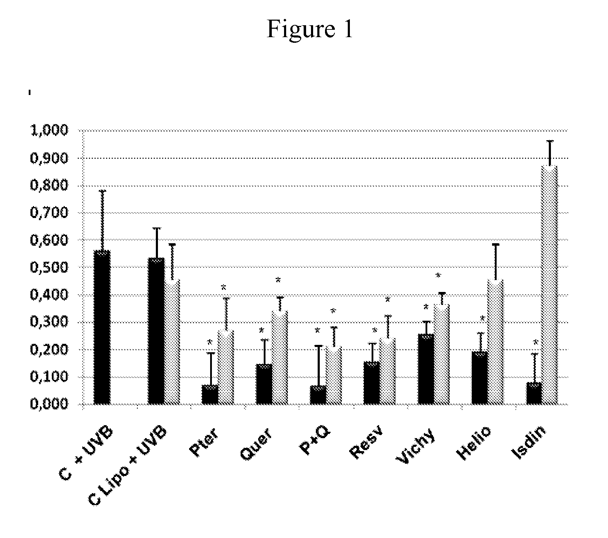

As shown in the **Example 10**, the determination of the skinfold thickness was carried out, the results being represented in **Figure 1**. As shown in **Figure 1**, the combination of PTER + QUER, and also PTER separately, are more efficient as photoprotector, when applied before the exposure to UVB, than the commercial creams: Vichy (VICHY Capital Soleil 50+ UVB+UVA), Heliocare (Heliocare ultra 90 UVB/UVA) and Isdin (ISDIN Extrem 40 high protection UVA and UVB). Those 3 commercial creams are used for comparison effects all along the present application specification. Also, along present application, RESV is also compared, in the same conditions assay, with PTER and PTER+QUER. RESV has been previously disclosed as chemoprotective agent of skin cancer (Moammir Hasan Aziz, Shannon Reagan-Shaw, Jianquiang Wu, B. Jack Longley and Nihal Ahmad; FASEB J. express article 10.1096 (2005): 1-18). In Fig. 1, PTER alone or the combination PTER+QUER show more efficacy for skin photoprotection either after or before UVB exposure than RESV.

Therefore either the combination of PTER + QUER, or PTER separately, could be used efficiently in the prevention of skin diseases induced by ultraviolet B radiation.

Moreover, **Figure 1** also shows that the combination of PTER + QUER is more efficient in the treatment of skin diseases, injuries or damages caused by radiation, when applied after the exposure to UVB, than the commercial creams (Vichy, Heliocare and Isdin). Therefore the combination of PTER + QUER could be used efficiently in the treatment of skin diseases, injuries or damages induced by ultraviolet B radiation.

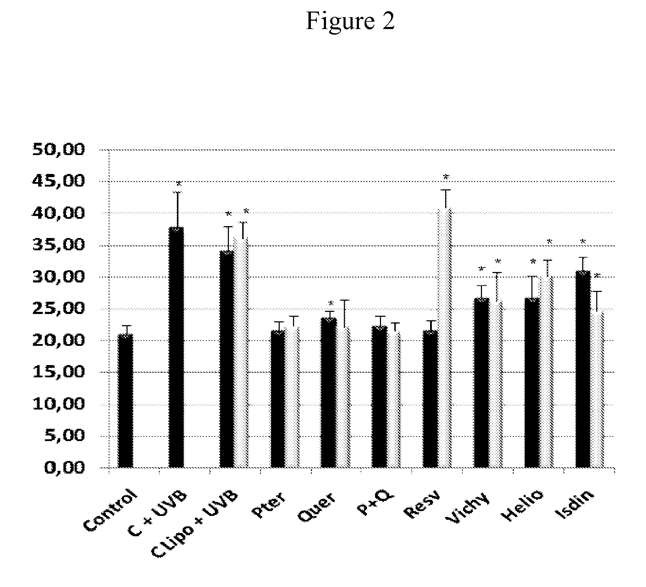

As shown in the **Example 11**, the measure of the thickness of the epidermis after irradiation (24 hours) was carried out, the results being represented in **Figure 2**. As shown in **Figure 2****,** the use of PTER separately is more efficient as photoprotector, when applied before the exposure to UVB, than the commercial creams (Vichy, Heliocare and Isdin). Therefore PTER separately could be used efficiently in the prevention of skin diseases induced by ultraviolet B radiation.

Moreover, **Figure 2** also shows that the combination of PTER + QUER, or PTER separately, are more efficient in the treatment of skin diseases, injuries or damages caused by radiation, when applied after the exposure to UVB, than the commercial creams (Vichy, Heliocare and Isdin). Therefore the combination of PTER + QUER, as well as PTER separately, could be used efficiently in the treatment of skin diseases, damages or injuries induced by ultraviolet B radiation.

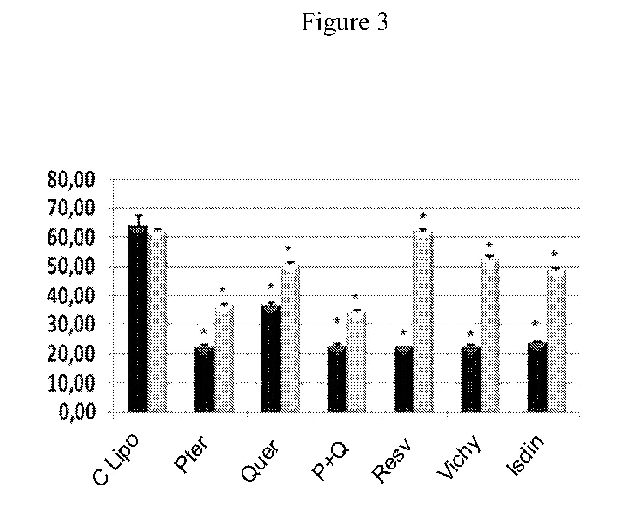

As shown in the **Example 12**, the measure of the thickness of the epidermis after irradiation (1 week) was carried out, the results being represented in **Figure 3**. As shown in **Figure 3**, the use of PTER separately is more efficient as photoprotector, when applied before the exposure to UVB, than the commercial creams (Vichy, Heliocare and Isdin). Therefore PTER separately could be used efficiently in the prevention of skin diseases, damages or injuries induced by ultraviolet B radiation.

Moreover, **Figure 3** also shows that the combination of PTER + QUER, and PTER separately, are more efficient in the treatment of skin diseases, injuries or damages caused by radiation, when applied after the exposure to UVB, than the commercial creams (Vichy, Heliocare and Isdin). Therefore the combination of PTER + QUER, as well as PTER separately could be used efficiently in the treatment of skin diseases, injuries or damages induced by ultraviolet B radiation.

Therefore, one embodiment of the present invention deals with the use as active compound of PTER, optionally in the form of acceptable salts thereof, for the manufacture of compositions for preventing and/or treating skin diseases, damages or injuries, particularly those caused by the exposure to radiation or associated to inflammatory processes.

Other embodiment of the present invention relates to the use of a combination of the active compounds PTER and QUER, or the combinations of acceptable salts thereof, for the manufacture of a topical composition for preventing and/or treating skin diseases, damages or injuries, particularly those caused by the exposure to radiation or associated to inflammatory processes.

A further embodiment of the invention relates to a composition, preferably a topical formulation, and more preferably in the form of liposomes, comprising the active compound PTER, or any acceptable salts thereof, for use in the prevention and/or treatment of skin diseases, damages or injuries, particularly those caused by the exposure to radiation or associated to inflammatory processes.

Another preferred embodiment of the invention relates to a topical composition, in the form of liposomes, comprising a combination of the two active compounds PTER and QUER, or any acceptable salts thereof, for use in the prevention and/or treatment of skin diseases, damages or injuries, particularly those caused by the exposure to radiation or associated to inflammatory processes.

One further embodiment of the present invention deals with the use of PTER, optionally in combination with QUER, or any acceptable salts thereof, for the manufacture of cosmetic compositions, preferably selected from sunscreens and aftersun formulations. More preferably those cosmetic formulations are for topical administration, preferably in the form of liposomes.

Other embodiment of the invention refers to a method for the preparation of PTER containing liposomes that comprises: dissolving the active compound PTER and, optionally, QUER, with lecithin, in dichloromethane, evaporating the solvent, adding PBS buffer, collecting and sonicating the liposomes formed, diluting 1:1 with an emulsion stabilizer, preferably a carbomer and more preferably Carbopol and optionally adding a preservative, preferably, Phenonip.

Carbopol is a carbomer. Carbomer is also a generic name for synthetic polymers of acrylic acid used as emulsion stabilizers or thickening agents in pharmaceuticals and cosmetic products. They may be homopolymers of acrylic acid, crosslinked with an allyl ether pentaerythritol, allyl ether of sucrose, or allyl ether of propylene.

Phenonip is a preservative clear liquid that works in a multitude of applications. It's unique in that it's soluble in oil and dispersible in water. This preservative was designed with cosmetics in mind and is especially effective when used in conjunction with oil based products but also works extremely well in aqueous solutions. This means that it will also work in products, like balms and salves, that contain only oils. Phenonip is also able to withstand a broader temperature range. Tests show that it can be heated to sterilization temperatures without degrading the quality of its preservative effects. It works in a wide ph range of 3 - 8. The usage rate varies from1% to 3% of weight of total formula (depending on the application).

Phenonip is composed of and should be labeled as: phenoxyethanol, methylparaben, ethylparaben, butylparaben, propylparaben.

The last embodiment of the invention refers to a method for the prevention and/or treatment of skin diseases, damages or injuries, particularly those caused by the exposure to radiation or associated to inflammatory processes, that comprises the administration of an effective amount of PTER, or any acceptable salt thereof, optionally in combination with QUER, or any acceptable salt thereof, to a subject in need of it.

### BRIEF DESCRIPTION OF THE FIGURES

**Figure 1**. It shows the skin fold thickness (mm) of mice treated with different compositions before (left column) or after (right column) being exposed to UVB. Commercial creams (Vichy, Heliocare -Helio- and Isdin). C+UVB= Control irradiated without any treatment; C Lipo+UVB= Control irradiated wherein only liposomes (without containing any active compound) applied on. RESV= Resveratrol; P+Q= PTER+QUER.
**Figure 2**. It shows the thickness (µm) of the epidermis treated with different compositions before (left column) or after (right column) being exposed to irradiation (24 hours). Commercial creams (Vichy, Heliocare -Helio- and Isdin). Control= No irradiation, no treatment; C+UVB= Control irradiated without any treatment; C Lipo+UVB= Control irradiated wherein only liposomes (without containing any active compound) applied on. RESV= Resveratrol; P+Q= PTER+QUER.
**Figure 3**. It shows the thickness (µm) of the epidermis treated with different compositions before (left column) or after (right column) being exposed to irradiation (1 week). Commercial creams (Vichy, Heliocare -Helio- and Isdin). C Lipo+UVB= Control irradiated wherein only liposomes (without containing any active compound) applied on. RESV= Resveratrol; P+Q= PTER+QUER.

In all Figures 1-3 the asterisk * shown in the bars histogram represents a difference statistically significant (P < 0.05), when compared with control values.

### EXAMPLES

### Example 1.

Studies for evaluating the capacity of the compositions of the invention of preventing skin diseases (such as skin cancer) induced by ultraviolet B radiation were carried out by acute irradiation at a dose of 360 mJ/cm².

### Example 2. Experimental animal model

Strain SKH-1 mice provided by Charles River Company were used as experimental animals. They are hairless immunocompetent mice. The experimental groups were formed with female mice aged 3 to 4 weeks as showed in **Table 2**. Each experimental group of photoprotection consisted of between 5 and 6 animals. In these studies, each experimental group consisted of 20 mice.

**Table 2**

| **GROUP** | **NAME** | **TREATMENT** | **UVB EXPOSURE** |
|---|---|---|---|
| I | Negative control | Liposomal vehicle | No |
| IIt | UVBa | Liposomal vehicle | 20 min after treatm. |
| IIa | UVBb | Liposomal vehicle | 20 min before treatm. |
| IIIt | RESa | RES liposomes | 20 min after treatm. |
| IIIa | RESb | RES liposomes | 20 min before treatm. |
| IVt | PTERa | PTER liposomes | 20 min after treatm. |
| IVa | PTERb | PTER liposomes | 20 min before treatm. |
| Vt | QUERa | QUER liposomes | 20 min after treatm. |
| Va | QUERb | QUER liposomes | 20 min before treatm. |
| VIt | (PTER+QUER)a | PTER+QUER liposomes | 20 min after treatm. |
| VIa | (PTER+QUER)b | PTER+QUER liposomes | 20 min before treatm. |
| VIIt | ISDINa | | 20 min after treatm. |
| VIIa | ISDINb | | 20 min before treatm. |
| VIIIt | HELICAREa | | 20 min after treatm. |
| VIIIa | HELIOCAREb | | 20 min before treatm. |
| IXt | VICHYa | | 20 min after treatm. |
| IXa | VICHYb | | 20 min before treatm. |

| | | | |
|---|---|---|---|
| b refers to"before" a refers to "after" | | | |

### Example 3. Protocol for exposure to UVB radiation.

To make the exposure to UVB a specially upgraded camera (Crosslinker equipped with 5 tubes which emit 80% of the radiation as UVB, with a maximum emission at 312 nm) was used.

UVB Acute exposure protocols were used: Mice underwent a single exposure of 360 mJ/cm2. The study was conducted at 24 hours after exposure. This protocol was used to evaluate the photoprotective effect of the compounds studied. With the same acutephase protocol mice from all groups were irradiated. Subsequently mice remained in the animal storage area for a week to see the evolution of the inflammatory response and edema.

### Example 4. Administration and dosage of the compounds under study

For the two studies a topical administration to the back of the mouse was conducted. The commercial sunscreens were applied directly on the mouse. The polyphenols in the study were administered in a liposomal preparation diluted 1:1 with Carbopol polymer which will be the vehicle for these liposomes. Each polyphenol dose administered was 10 µmol/mouse, as free acid.

### Example 5. Preparation of liposomes

Regardless of the polyphenol(s) the methodology of preparation of liposomes were equally used: soy lecithin; dichloromethane as organic solvent in the case of pterostilbene and/or quercetin, and ethyl ether as organic solvent for resveratrol due to its inability to dissolve in dichloromethane. The evaporation of organic solvent was performed using a rotary evaporator connected to vacuum and a temperature of 40 °C. After removal of organic solvent, liposomes were resuspended in PBS diluted to 1 mM. In the final liposome concentration, before adding the Carbopol, the concentration of each polyphenol was of 20 µM polyphenols as determined by HPLC / MS-MS.

The steps for preparing the liposomes can be summarized as follows:
1. Weight polyphenols and lecithin

| **PTER** | **QUER** | **RESV** | **LECITHIN** |
|---|---|---|---|
| **0,512gr** | **0,676gr** | **0,458gr** | **2,376** |

2. Dissolve the polyphenol and lecithin in 40ml of dichloromethane (in the case of pterostilbene and/or quercetin), or 140 ml of ethyl ether (in the case of resveratrol).
3. Introduce the solution into a rotary evaporator flask and attached to the device. Once all the solvent evaporated cut the expected vacuum and wait for 15 min. to eliminate all traces of solvent which may have become.
4. Add 20ml of diluted PBS (1mM) in the rotary evaporator flask and re-attach the device but this time without vacuum.
5. The liposomes formed are collected in a 50 ml tube.
6. Sonication of liposomes (6 seconds, 25 cycles, with an interval of 3 seconds between each cycle; *high intensity*)*.*
7. Dilution 1:1 with Carbopol (final concentration of 10 µmoles/200 µl)
8. To avoid possible contamination is added Phenonip (6ml of Phenonip per 1000 ml of preparation).

Control liposomes were made in the same manner as those of pterostilbene and quercetin, but only contain lecithin (2.376g).

### Example 6. Determination ofpolyphenol concentration in liposomes

To ensure that the liposome preparations contained the expected amounts of polyphenols were conducted periodic determinations by HPLC / MS-MS.

### Example 7. Parameters indicators of skin damage

1. Edema formation: skinfold thickness was determined with a caliper 24 hours after the first exposure.
2. Study of the thickness of the epidermis after exposure to acute irradiation (360mj/cm² 24h hours and 7 days after irradiation).
3. Histological studies: measuring, in the pictures obtained from histological sections, the thickness of the epidermis.

### Example 8. Histological studies

For studies of acute exposure skin samples were taken from the back of the mouse. These samples were fixed with 4% paraformaldehyde (24 hours) and embedded in paraffin. Following cutting the samples were stained with hematoxylin and eosin.

### Example 9. Sacrifice and collection of samples

The sacrifice was carried out by cervical dislocation. Subsequently, 20 µl of blood were taken and added to 80 µl ofN-ethylmaleimide (NEM).

After incubation for about 10 min. were added 100 µl perchloric acid (PCA) 4%. After vigorous shacking samples were preserved in ice. Finally all samples were centrifuged at 13000 rpm for 15 minutes at 4°C. Supernatants were collected and stored at -20 °C until analysis of the GSH (reduced form of glutathione) / GSSG (oxidized form of glutathione) ratio. The GSH/GSSG ratio is a parameter that reflects the intracelular redox status, and the intracellular thiol redox staus in particular. Therefore changes in this ratio are used to evaluate oxidative stress.

Four samples were taken from skin of the hindquarters. One for histology (was fixed overnight in 4% formaldehyde) and 3 were frozen with liquid nitrogen for biochemical analysis.

### Example 10. Determination of skin fold 24 h after irradiation

**Fig. 1** shows the results obtained in measuring the thickness of the skin of the hindquarters mice (where the various treatments have been applied). The results were statistically analyzed by ANOVA and contrasted by the Tukey test and grouped according to the significant differences. Standard deviations are incorporated in **Fig. 1**. As shown in **Figure 1**, the combination of PTER + QUER and also PTER separately, are more efficient as photoprotector, when applied before the exposure to UVB, than the commercial creams (Vichy, Heliocare and Isdin) and that RESV. Therefore either the combination of PTER + QUER or PTER separately could be used efficiently in the prevention of skin diseases induced by ultraviolet B radiation.

Moreover, **Figure 1** also shows that the combination of PTER + QUER or PTER separately are more efficient for the treatment of skin diseases, damages or injuries due to radiation, when applied after the exposure to UVB, than the commercial creams (Vichy, Heliocare and Isdin) and that RESV. Therefore the combination of PTER + QUER could be used efficiently in the treatment of skin diseases, damages or injuries induced by ultraviolet B radiation.

### Example 11. Measuring the thickness of the epidermis after irradiation (24 hours)

The evaluation of skin thickness was carried out by using computer software (IMAGE J). Results obtained were statistically analyzed by ANOVA and contrasted by the Tukey test and grouped according to the significant differences. As shown in **Figure 2**, the use of PTER alone is more efficient as photoprotector, when applied before the exposure to UVB, than the commercial creams (Vichy, Heliocare and Isdin). Therefore PTER alone could be used efficiently in the prevention of skin diseases, injuries or damages induced by ultraviolet B radiation.

Moreover, **Figure 2** also shows that the combination of PTER + QUER is also more efficient for the treatment of skin diseases, damages or injuries caused by radiation, when applied after the exposure to UVB, than the commercial creams (Vichy, Heliocare and Isdin) and that RESV. Therefore the combination of PTER + QUER, as well as PTER separately, could be used efficiently in the treatment of skin diseases, damages or injuries induced by ultraviolet B radiation. Control in this experiment means no radiation and no treatment.

### Example 12. Measuring the thickness of the epidermis after irradiation (1 week)

The evaluation of skin thickness was carried out by using computer software (IMAGE J). Results obtained by ANOVA were contrasted by the Tukey test and grouped according to the significant differences. As shown in **Figure 3**, the use of PTER separately is more efficient as photoprotector, when applied before the exposure to UVB, than the commercial creams (Vichy, Heliocare and Isdin). Therefore PTER separately could be used efficiently in the prevention of skin diseases, damages or injuries induced by ultraviolet B radiation.

Moreover, **Figure 3** also shows that the combination of PTER + QUER is more efficient too, for the treatment of skin diseases, damages or injuries caused by radiation, when applied after the exposure to UVB, than the commercial creams (Vichy, Heliocare and Isdin) and that RESV. Therefore the combination of PTER + QUER, as well as PTER separately could be used efficiently in the treatment of skin diseases induced by ultraviolet B radiation.

## Claims

1. Use of PTER or any acceptable salt thereof, optionally in combination with QUER or any acceptable salt thereof, for preventing and/or treating skin diseases, damages or injuries.

2. Use, according to claim 1, **characterized in that** the skin diseases, damages or injuries are associated to inflammatory processes.

3. Use, according to claim 1, **characterized in that** the skin diseases, damages or injuries are caused by the exposure to radiation.

4. Use of PTER or any acceptable salt thereof, optionally in combination with QUER or any acceptable salt thereof, for the manufacture of a medicament for preventing and/or treating skin diseases, damages or injuries.

5. Use according to claim 4 wherein the medicament is a pharmaceutical composition for topical administration

6. Use according to claim 5 wherein the pharmaceutical composition for topical administration is formulated in the form of liposomes.

7. Use of PTER or any acceptable salt thereof, optionally in combination with QUER or any acceptable salt thereof, for manufacturing cosmetics.

8. Use according to claim 7 wherein the cosmetic is applied to the skin by topical administration.

9. Use according to claim 8 wherein the cosmetic for topical administration is formulated in the form of liposomes.

10. Use according to any of the claims 7 to 9 wherein the cosmetic is in the form of sunscreen or after-sun formulations.

11. Pharmaceutical composition comprising PTER as active compound or any acceptable salt thereof, optionally in combination with QUER or any acceptable salt thereof, for topical administration.

12. Pharmaceutical composition, according to claim 11, further comprising any acceptable excipient, vehicle or carrier, suitable for topical administration.

13. Pharmaceutical composition, according to any of the claims 11 or 12, **characterized by** being formulated in the form of liposomes.

14. Cosmetic composition comprising PTER as active compound or any acceptable salt thereof, optionally in combination with QUER or any acceptable salt thereof, for topical administration.

15. Cosmetic composition, according to claim 14, further comprising any acceptable excipient, vehicle or carrier, suitable for topical administration.

16. Cosmetic composition, according to any of the claims 14 or 15, **characterized by** being formulated in the form of liposomes.

17. Cosmetic composition according to any of the claims 14 to 16 **characterized by** being in the form of sunscreen or aftersun formulations.

18. Process for manufacturing liposome formulations that comprises:
a) Dissolving the active compound PTER, or any acceptable salt thereof, and lecithin in an organic solvent.
b) Evaporating the solvent.
c) Adding PBS buffer.
d) Collecting and sonicating the liposomes formed.
e) Diluting 1:1 with an emulsion stabilizer, preferably a carbomer and more preferably Carbopol
f) Optionally adding a preservative, preferably Phenonip

19. Process, according to claim 18, wherein QUER, or any acceptable salt thereof, is also optionally dissolved in step (a).

20. Method for the prevention and/or treatment of skin diseases, damages or injuries that comprises the administration of PTER or any acceptable salt thereof, optionally in combination with QUER or any acceptable salt thereof, to a subject in need of it.

21. Method according to claim 20 that comprises the administration of an effective amount of the composition of claims 11 to 13 to a subject in need of it.

22. Method, according to claims 20 or 21, wherein the skin diseases, damages or injuries are associated to inflammatory processes.

23. Method, according to claims 20 or 21, wherein the skin diseases, damages or injuries are caused by radiation.
